# EUROPÄISCHE PATENTSCHRIFT

(11) **EP 2 857 051 B1**
(45) Veröffentlichungstag und Bekanntmachung des Hinweises auf die Patenterteilung: **13.07.2016**
(21) Anmeldenummer: 14003174.1
(22) Anmeldetag: 13.09.2014
(51) Int. Cl.: A61M 1/00

(54) **Einrichtung zur Behandlung von intraluminalen Verletzungen des Gastro-Intestinal-Trakts**
Device for the treatment of internal injury to the gastro-intestinal tract
Dispositif de traitement de lésions intraluminales du tractus gastro-intestinal

(30) Priorität: 03.10.2013 AT 7642013
(43) Veröffentlichungstag der Anmeldung: 08.04.2015
(73) Patentinhaber: A.M.I. Agency for Medical Innovations GmbH, 6800 Feldkirch (AT)
(72) Erfinder: Egle, Walter, 6842 Koblach (AT)
(74) Vertreter: Hofmann, Ralf U.

(56) Entgegenhaltungen:
- WO-A1-2012/123414
- DE-T2- 60 126 748
- FR-A1- 2 350 849
- US-A- 3 905 361
- US-A- 6 027 478
- US-A1- 2005 261 615

## Beschreibung

Die Erfindung bezieht sich auf eine Einrichtung zur Behandlung von intraluminalen Verletzungen des Gastro-Intestinal-Trakts, umfassend einen Rohrkörper mit einem den Rohrkörper in Richtung seiner Längserstreckung durchsetzenden flüssigkeitsdichten Durchgangskanal und ein an der äußeren Oberfläche des Rohrkörpers angeordnetes poröses Material, aus welchem vom porösen Material aufgenommenes Fluid mittels einer Drainageleitung absaugbar ist.

Zur Behandlung von inneren Wunden des Gastro-Intestinal-Trakts kann herkömmlicherweise die endoskopische Vakuumtherapie eingesetzt werden. Unter Zuhilfenahme von Endoskopen werden offenporige Polyurethanschwammkörper im Lumen in Defekthöhe platziert. Über einen daran angenähten Drainageschlauch wird ein Unterdruck erzeugt, wodurch sich die Schwammoberfläche an die Wundoberfläche ansaugt und eine effektive Wunddrainage erfolgt.

Dokument WO2012123414 offenbart ein Vakuumsystem und Endoskopie-Anordnung für endoskopische Vakuumtherapie.

Eine Einrichtung zur Durchführung der Vakuumtherapie in tieferliegenden Bereichen, beispielsweise des Gastro-Intestinal-Trakts geht aus der DE 10 2012 023 061 A1 hervor. Ein Drainageschlauch ist zumindest abschnittsweise von einem porösen Material umgeben. Der Drainageschlauch besitzt Perforationsöffnungen, durch welche das poröse Material mit einem Unterdruck beaufschlagbar ist und vom porösen Material aufgenommenes Fluid absaugbar ist. Bei dieser Einrichtung muss der Abschnitt des Gastro-Intestinal-Trakts, in welchem die Einrichtung eingesetzt wird, frei von Inhaltsstoffen sein. Auch ist die Sicherheit gegen ein Verrutschen der Einrichtung im Gastro-Intestinal-Trakt eingeschränkt.

Aus der EP 1 633 279 B1 geht eine Einrichtung zur Behandlung von Undichtigkeiten von chirurgischen Nahtverbindungen (Anastomosen) im Magen-Darm-Trakt hervor. Diese stellen eine gefährliche Komplikation nach Operationen im Bauchraum dar.

Zur Behandlung solcher Undichtigkeiten wird ein Stent zur Einführung in den Gastro-Intestinal-Trakt bereitgestellt, welcher einen radial expandierbaren und in Längsrichtung offenen Rohrkörper, ein den Rohrkörper umhüllendes poröses formbares Material und eine in das Material eingebrachte Drainage umfasst, die zum Anlegen eines Unterdrucks ausgebildet ist, wobei das Lumen des Hohlkörpers gegenüber dem Material luft- und wasserdicht ist. Nach dem Einsetzen und der Expansion des Stents, sodass dieser an der Wand des Gastro-Intestinal-Trakts anliegt, wird die Drainageleitung mit einem Unterdruck beaufschlagt, wodurch die Wand des Gastro-Intestinal-Trakts zirkulär an den Stent angesaugt wird und damit ein Austritt von Flüssigkeit aus dem Hohlorgan verhindert wird. Zudem wird Flüssigkeit, die sich im Bereich der Undichtigkeit angesammelt hat, durch die Drainageleitung abgeführt. Im angelegten Zustand des Stents können Inhaltsstoffe des Gastro-Intestinal-Trakts durch dessen Durchgangskanal durchtreten.

Die US 6,994,095 B2 zeigt eine nicht gattungsgemäße Einrichtung zur Behandlung von Fettleibigkeit. Mittels dieser Einrichtung wird eine Magenöffnung, insbesondere der Pylorus, teilweise oder zeitweise verschlossen. An einem dazwischen sich erstreckenden Tragteil sind Ringballone angebracht, von denen einer im Einsatzzustand der Einrichtung innerhalb des Magens und der andere außerhalb des Magens im anschließenden Dünndarm platziert ist. Durch den zwischen den Ringballonen liegenden Ringmuskel des Pylorus ist eine Sicherung gegen ein Verrutschen formschlüssig gegeben. Das Tragteil kann einen Durchgangskanal zum (reduzierten) Durchtritt von Speisebrei aufweisen.

Aus der US 4,781,677 A ist eine nicht gattungsgemäße Einrichtung in Form eines Katheters zur Einführung in den Gallengang bekannt, um eine Untersuchung und/oder Behandlung der Gallenblase, insbesondere zur Entfernung von Gallensteinen, durchzuführen. Die Einrichtung umfasst zwei befüllbare Ballonelemente, die über einen zwischen ihnen sich erstreckenden Schlauch, der mehrere Durchgangskanäle besitzt, verbunden sind. Im Einsatz dichten die beiden Ballonelemente den Gallengang beidseitig der Abzweigung des zur Gallenblase führenden ductus cysticus ab. Im zwischen dem ersten und dem zweiten Ballonelement liegenden Bereich des Schlauchs mündet einer der Durchgangskanäle. Dieser dient zum Einführen von Kontrastmittel zur Untersuchung der Gallenblase und Einführung einer Behandlungsflüssigkeit zum Auflösen von Gallensteinen sowie zum Abführen der Behandlungsflüssigkeit. Der Gallengang weist einen nur geringen Durchmesser von wenigen Millimetern auf und die Ballonelemente sind auf eine damit verbundene nur geringe Größe expandierbar.

Aufgabe der Erfindung ist es eine Einrichtung der eingangs genannten Art bereitzustellen, die bei einer einfachen und zuverlässigen Ausbildung eine vorteilhafte Behandlung eines Patienten ermöglicht und in einfacher Weise am Ort der Verletzung anbringbar und wieder entfernbar ist. Erfindungsgemäß gelingt dies durch eine Einrichtung mit den Merkmalen des Anspruchs 1.

Bei der Einrichtung gemäß der Erfindung ist das poröse Material nur über einen Teil der Längserstreckung des Rohrkörpers an der äußeren Oberfläche des Rohrkörpers angeordnet. Beidseitig dieses Teils der Längserstreckung des Rohrkörpers wird der Rohrkörper jeweils von einem elastisch dehnbaren Ballonelement umgeben. Ein jeweiliges Ballonelement, vorzugsweise in Verbindung mit der von ihm umgebenen Wand des Rohrkörpers, bildet einen Ringballon, dessen Außendurchmesser durch Befüllen mit einem Befüllmedium vergrößerbar ist. Ein jeweiliges Ballonelement weist eine vorgeformte Wölbung (=Krümmung) auf, welche es also im drucklosen Zustand (d.h. der Innendruck ist gleich dem Außendruck) einnimmt. Die das jeweilige Ballonelement bildende Membrane ist also im drucklosen Zustand zur Außenseite hin (also in die von der Längsmittelachse des Rohrkörpers weggerichtete Richtung) konvex gekrümmt. Durch diese Wölbung schließt der vom jeweiligen Ballonelement, vorzugsweise in Verbindung mit der von ihm umgebenden Wand des Rohrkörpers, gebildete Ringballon im drucklosen Zustand einen Hohlraum ein. Vorzugsweise weist ein jeweiliges Ballonelement im drucklosen Zustand einen größten Außendurchmesser auf, der mindestens 50% größer ist als der Außendurchmesser des Rohrkörpers in seinem Abschnitt, über welchen der vom jeweils betreffenden Ballonelement umgeben ist.

Im Einsatz der Einrichtung werden die Ringballons, die von den Ballonelementen, vorzugsweise in Verbindung mit dem von ihnen jeweils umgebenen Abschnitt der Wand des Rohrkörpers, gebildet werden, nach der erfolgten Platzierung der Einrichtung mit dem Füllmedium befüllt, bis die geeignete Ausdehnung der Ballonelemente erreicht ist. Im Weiteren wird ein Unterdruck an die Drainageleitung angelegt, sodass die Innenseite des Gastro-Intestinal-Trakts, in welchem sich die intraluminale Verletzung des Gastro-Intestinal-Trakts befindet über einen zwischen den Ballonelementen liegenden Abschnitt an die Einrichtung angesaugt wird. Damit wird eine sehr zuverlässige Sicherung der Einrichtung gegen ein Verrutschen im Gastro-Intestinal-Trakt erreicht. Im Weiteren wird ein sehr zuverlässiger Schutz der verletzten Stelle des Gastro-Intestinal-Trakts gegenüber den den Gastro-Intestinal-Trakt passierenden mehr oder weniger flüssigen Inhaltsstoffen erreicht.

Die Ausbildung der Ballonelemente mit der vorgeformten Wölbung trägt hierbei erheblich zur Sicherheit der Einrichtung bei. Der für die Ausdehnung der Ballonelemente auf den gewünschten Umfang erforderliche Druck des Befüllmediums kann dadurch wesentlich geringer gehalten werden als ohne eine solche vorgeformte Wölbung. Für das Einführen der Einrichtung kann der vom jeweiligen Ringballon eingeschlossene Hohlraum durch die mit diesem Hohlraum kommunizierende Befüllleitung evakuiert werden. Dadurch legt sich das Ballonelement zumindest teilweise unter Faltenbildung an die äußere Oberfläche des Rohrkörpers an. Der äußere Durchmesser der Einrichtung wird dadurch beim Einführen minimiert.

Das an der äußeren Oberfläche des Rohrkörpers angeordnete poröse Material ist vorteilhafterweise zur Außenseite der Einrichtung hin von einer mit Durchtrittsöffnungen versehenen Folie abgedeckt, wie dies von der Vakuumtherapie her bekannt ist. Dadurch wird einem Einwachsen von Körpergewebe in das poröse Material entgegengewirkt.

In einem vorteilhaften Verfahren zur Herstellung einer erfindungsgemäßen Einrichtung werden der Rohrkörper und die Ballonelemente oder zumindest ein Teil, welches eine Schicht des Rohrkörpers bildet, und die Ballonelemente als gemeinsames Spritzgussteil ausgebildet. Um eine ausreichende Elastizität der Ballonelemente und eine ausreichende Stabilität des Rohrkörpers bzw. des zumindest eine Schicht des Rohrkörpers bildenden Teils auszubilden, kann hierbei dieses Spritzgussteil entsprechend unterschiedliche Wandstärken aufweisen. Die die Ballonelemente bildenden Abschnitte dieses Spritzgussteils, welche an die beiden Längsenden des Spritzgussteils anschließen, werden nach dem Spritzen des Spritzgussteils umgestülpt. Damit kommen die die Ballonelemente bildenden Abschnitte des Spritzgussteils über den äußeren Oberflächen von Abschnitten des Spritzgussteils zu liegen, welche an die Längsenden des Spritzgussteils anschließen. Die umgestülpten, die Ballonelemente bildenden Abschnitte des Spritzgussteils werden dann endseitig mit der äußeren Oberfläche des Rohrkörpers verbunden, insbesondere durch Verkleben oder Verschweißen.

Weitere Vorteile und Einzelheiten der Erfindung werden im Folgenden anhand der beiliegenden Zeichnung erläutert. In dieser zeigen:
Fig. 1 eine Seitenansicht eines Ausführungsbeispiels einer erfindungsgemäßen Einrichtung;
Fig. 2 eine Schrägsicht der Einrichtung von Fig. 1;
Fig. 3 einen Längsmittelschnitt durch die Einrichtung von Fig. 1;
Fig. 4 und 5 Darstellungen entsprechend Fig. 2 und 3 im expandierten Zustand der Ballonelemente;
Fig. 6 und 7 Darstellungen entsprechend Fig. 2 und 3 im evakuierten Zustand der Ballonelemente;
Fig. 8, 9 und 10 eine Seitenansicht, einen Längsmittelschnitt und eine Schrägsicht eines Spritzgussteils für die Herstellung einer erfindungsgemäßen Einrichtung;
Fig. 11, 12 und 13 Darstellungen entsprechend Fig. 8, Fig. 9 und Fig. 10 eines weiteren Herstellungsschrittes der Einrichtung;
Fig. 14 eine schematische Darstellung der erfindungsgemäßen Einrichtung im Einsatz.

Ein Ausführungsbeispiel einer Einrichtung gemäß der Erfindung ist in den Figuren dargestellt. Die Einrichtung besitzt einen Rohrkörper 1 mit einer Längsmittelachse 2. Der Rohrkörper 1 wird von einem flüssigkeits- und luftdichten Durchgangskanal 3 axial, d.h. in Richtung seiner Längserstreckung vollständig durchsetzt. Der Durchgangskanal 3 mündet also an beiden Längsenden des Rohrkörpers 1 (=ist zu beiden Längsenden hin offen).

Der Rohrkörper 1 wird von einem ersten und einem zweiten Ballonelement 4, 5 umgeben, die in axialer Richtung des Rohrkörpers 1 voneinander beabstandet sind. Im Ausführungsbeispiel schließen die Ballonelemente 4, 5 direkt an die axialen Enden des Rohrkörpers 1 an. Die Ballonelemente 4, 5 bilden zusammen mit den äußeren Oberflächen der von ihnen umgebenen Abschnitte des Rohrkörpers 1 jeweils einen Ringballon.

Im Bereich zwischen den beiden Ballonelementen 4, 5 ist ein poröses Material 6 an der äußeren Oberfläche des Rohrkörpers 1 angebracht, insbesondere angeklebt, welches den Rohrkörper 1 vorzugsweise vollumfänglich umgibt. Das poröse Material 6 ist offenporig ausgebildet. Somit ist vom porösen Material 6 Fluid aufnehmbar und wieder abgebbar (vorzugsweise mit einem Volumen von mindestens 20% des Volumens des porösen Materials 6 im unkomprimierten Zustand). Dem porösen Material 6 ist eine Drainageleitung 7 zugeordnet, die im oder am porösen Material 6 mündet. Mittels dieser Drainageleitung 7 kann vom porösen Material 6 aufgenommenes Fluid aus diesem abgesaugt werden. Im Ausführungsbeispiel wird die Drainageleitung 7 von einem Röhrchen gebildet, welches in den Durchgangskanal 3 hinein und durch einen Teil von diesem verläuft und in eine Bohrung im Rohrkörper 1 ragt bzw. diese durchsetzt.

Wie in den Figuren dargestellt, ist es bevorzugt, dass der Rohrkörper 1 über den Teil seiner Längserstreckung, über welchen das poröse Material 6 an ihm angebracht ist, vollständig vom porösen Material 6 abgedeckt ist (und nicht etwa nur bereichsweise).

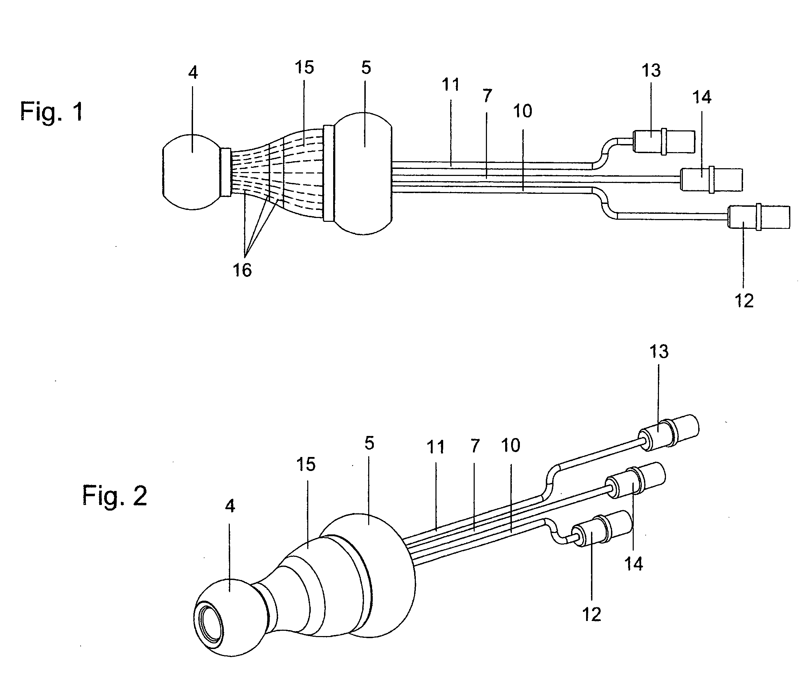

Die Ballonelemente 4, 5 weisen eine vorgeformte Wölbung auf, d.h. die Membrane, von welchen die Ballonelemente 4, 5 ausgebildet werden, sind zur Außenseite hin (also in die von der Längsmittelachse 2 weggerichtete Richtung) konvex gekrümmt, und zwar ohne dass eine Druckdifferenz auf beiden Seiten dieser Membrane vorliegt. Dadurch schließen die Ballonelemente 4, 5 zusammen mit dem jeweiligen Abschnitt des Rohrkörpers 1, den sie umgeben, im drucklosen Zustand jeweils einen Hohlraum 8, 9 ein (und liegen nicht etwa durchgehend an der äußeren Oberfläche des Rohrkörpers 1 an). Die radiale Erstreckung dieses Hohlraums beträgt vorzugsweise mindestens 3mm. Dieser drucklose Zustand, d.h. der Innendruck des Hohlraums 8, 9 ist gleich dem Außendruck, ist in den Fig. 1 bis 3 dargestellt.

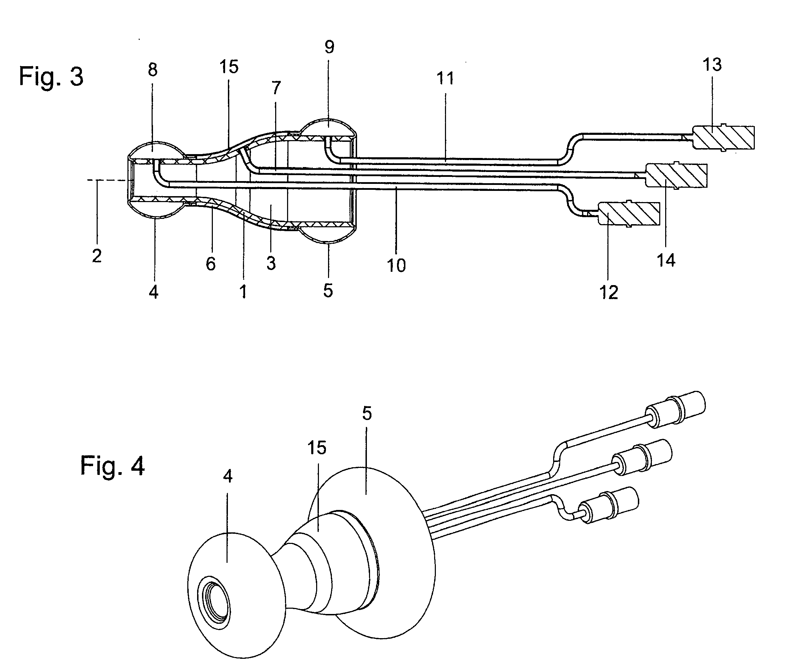

Den Ballonelementen 4, 5 ist jeweils eine Befüllleitung 10, 11 zugeordnet, die mit dem jeweiligen Hohlraum 8, 9 kommuniziert. Im Ausführungsbeispiel wird eine jeweilige Befüllleitung 10, 11 von einem Röhrchen gebildet, welches in den Durchgangskanal 3 hinein und durch einen Teil des Durchgangskanals 3 verläuft. Das Röhrchen ragt im Weiteren in eine Bohrung im Rohrkörper 1 oder durchsetzt diese und mündet in den jeweiligen Hohlraum 8, 9. In derjeweiligen Befüllleitung 10, 11 ist ein Ventil 12, 13 angeordnet, welches in den Fig. 3, 5 und 7 nur schematisch dargestellt ist. Im Ausführungsbeispiel sind die Ventile 12, 13 an den Enden der Befülleitung 10, 11 angeordnet und über eine geeignete Flanscheinrichtung, beispielsweise ein Luer-Lock, mit einer Einrichtung zum Befüllen und/oder Evakuieren des jeweiligen Hohlraums 8, 9 verbunden, beispielsweise einer Spritze. Diese Flanscheinrichtungen könnten auch getrennt von den Ventilen 12, 13 vorgesehen sein, sodass zwischen dem jeweiligen Ventil 12, 13 und der zugeordneten Flanscheinrichtung ein Abschnitt der jeweiligen Befüllleitung 10, 11 verläuft.

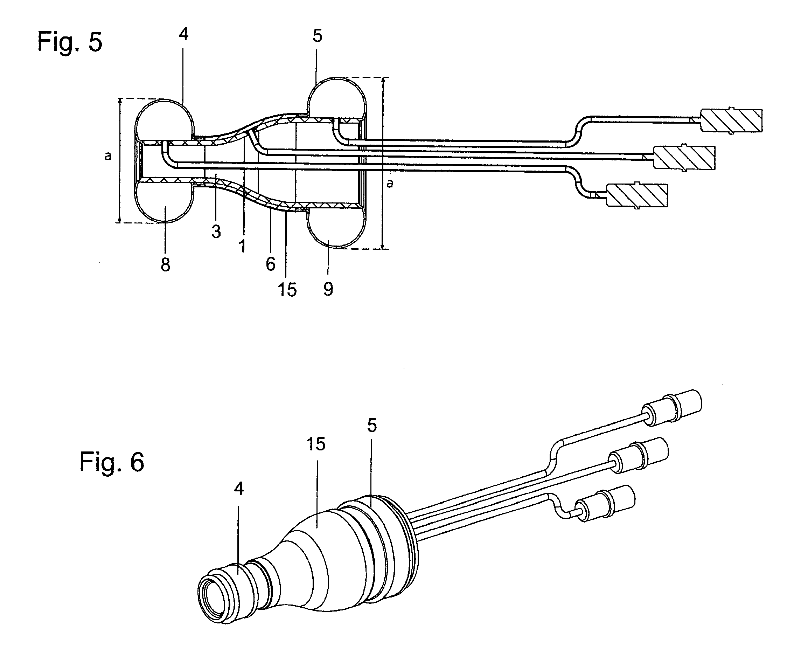

Wenn der jeweilige Hohlraum 8, 9, den das jeweilige Ballonelement 4, 5 begrenzt, ausgehend vom drucklosen Zustand mit einem Füllmedium befüllt wird, sodass ein Überdruck ausgebildet wird, so dehnt sich das jeweilige Ballonelement 4, 5 aus, wobei sich sein größter Außendurchmesser a vergrößert. Dieser expandierte Zustand der Ballonelemente 4, 5 ist in den Fig. 4 und 5 dargestellt.

Das Befüllmedium kann ein Gas, beispielsweise Luft oder Stickstoff, oder eine Flüssigkeit, beispielsweise Wasser oder eine Salzlösung, sein. Eine Befüllung mit einem Gas hat den Vorteil, dass die Ballonelemente 4, 5 komprimierbar bleiben.

Die Ventile 12, 13 können in Form von selbstschließenden Ventilen ausgebildet sein, welche schließen, wenn die Einrichtungen zum Befüllen von den mit diesen Ventilen integriert ausgebildeten Flanscheinrichtungen getrennt werden, oder es kann sich um entsperrbare Rückschlagventile handeln.

Die Drainageleitung 7 ist mit einer (in den Figuren nicht dargestellten) Absaugeinrichtung verbindbar, um einen Unterdruck in der Drainageleitung 7 zu erzeugen. Wenn nach dem Implantieren der Einrichtung, welches mit einem Befüllen der Ballonelemente 4, 5 durch die Befüllleitungen 10, 11 und Evakuieren des zwischen den Ballonelementen 4, 5 liegenden Bereichs durch die Drainageleitung 7 einhergeht, nur noch eine relativ geringe Menge an Fluid durch die Drainageleitung 7 abzusaugen ist, so muss das Absaugen durch die Drainageleitung 7 eventuell nicht permanent erfolgen, und die Drainageleitung 7 kann nach ihrer Evakuierung zumindest zeitweise verschlossen werden. Hierzu ist im Ausführungsbeispiel ein Ventil 14 vorhanden, von dem die Drainageleitung 7 verschließbar ist. Beispielsweise ist das Ventil 14 wie dargestellt am Ende der Drainageleitung 7 angeordnet. Das Ventil 14 kann dann mit einer Flanscheinrichtung, beispielsweise einem Luer-Lock zum Anschluss an eine Absaugeinrichtung, beispielsweise eine Spritze, eine Einheit bilden. Eine solche Flanscheinrichtung zum Anschluss einer Absaugeinrichtung kann auch vom Ventil 14 getrennt ausgebildet sein, sodass ein Abschnitt der Drainageleitung 7 zwischen dem Ventil 14 und der Flanscheinrichtung verläuft. Wenn die Drainageleitung 7 permanent abgesaugt wird, kann das Ventil 14 auch entfallen.

Beim Ventil 14 kann es sich um ein selbstschließendes Ventil handeln, welches schließt, wenn die Absaugeinrichtung von der mit diesem Ventil integriert ausgebildeten Flanscheinrichtung getrennt wird, oder es kann sich um ein entsperrbares Rückschlagventil handeln.

Das poröse Material 6 ist außen von einer Folie 15 umgeben. Diese ist mit einer Vielzahl von Durchtrittsöffnungen 16 versehen, welche der Einfachheit halber nur in Fig. 1 angedeutet sind. Beispielsweise können die Durchtrittsöffnungen 16 von Schnitten gebildet werden, welche in die Folie 15 eingebracht sind. Die Folie 15 könnte auch in anderer Weise perforiert ausgebildet sein.

Abgesehen von den Durchtrittsöffnungen 16 wird die äußere Oberfläche des porösen Materials 6 vollständig von der Folie 15 abgedeckt.

Beispielsweise kann die Folie 15 aus Silikon bestehen, auch Folien aus anderen körperverträglichen Materialien, beispielsweise aus PE, können eingesetzt werden.

Das poröse Material 6 kann beispielsweise von einem Silikonschaumstoff gebildet werden. Auch andere Schaumstoffe können eingesetzt werden, beispielsweise geschäumtes Polyurethan.

Der zwischen den Ballonelementen 4, 5 liegende Bereich des Rohrkörpers 1 ist vorzugsweise zumindest im Wesentlichen vollständig vom porösen Material 6 umgeben. D.h. die Ballonelemente 4, 5 schließen beidseitig direkt an das poröse Material 6 an oder weisen einen Abstand von diesem auf, der zumindest weniger als 20% des Abstands zwischen den beidseitig des porösen Materials 6 liegenden Ballonelementen 4, 5 beträgt. Im Ausführungsbeispiel sind Endabschnitte der die Ballonelemente 4, 5 bildenden Membranen an den einander zugewandten Seiten der Ballonelemente 4, 5 mit der äußeren Oberfläche des Rohrkörpers 1 verklebt und das poröse Material schließt unmittelbar an die Verbindungsbereiche der Membranen mit dem Rohrkörper 1 an. Die Folie 15 erstreckt sich über das poröse Material 6 und über die Endabschnitte der Membranen, über welche diese mit dem Rohrkörper 1 verbunden sind.

Anstelle einer Verbindung über eine Verklebung könnte beispielsweise auch eine Verbindung der Endabschnitte der Membranen mit dem Rohrkörper 1 über Verschweißungen erfolgen, wenn der Rohrkörper 1 und die Membranen aus einem hitzeverschweißbaren Material bestehen.

Im Ausführungsbeispiel ändert sich der Durchmesser des Rohrkörpers über den Teil seiner Längserstreckung, über welchen das poröse Material 6 an seiner äußeren Oberfläche angebracht ist. Damit weist er im Bereich, in welchem er vom ersten Ballonelement 4 umgeben ist, einen anderen, hier geringeren, Außendurchmesser auf, als im Bereich, in welchem er vom zweiten Ballonelement 5 umgeben ist. Eine solche Ausbildung ist vorteilhaft, wenn das Hohlorgan, in welchem die erfindungsgemäße Einrichtung eingesetzt wird, ihren Innendurchmesser über den Abschnitt, in welchem die Einrichtung angeordnet wird, ändert. In anderen Anwendungsfällen, wenn der Innendurchmesser des Hohlorgans gleichbleibt, kann der Rohrkörper im Bereich des ersten Ballonelements 4 und im Bereich des zweiten Ballonelements 5 gleiche Außendurchmesser aufweisen.

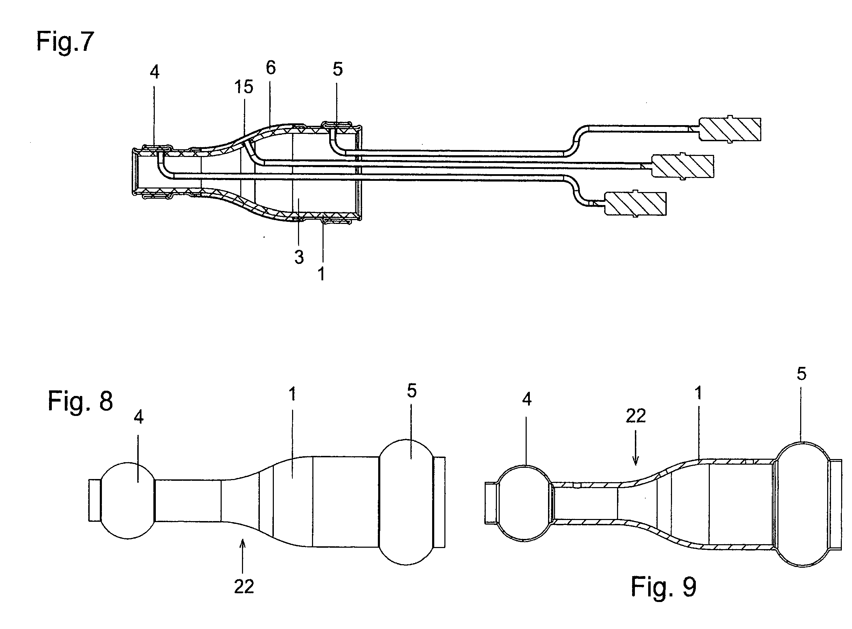

Zum Implantieren der Einrichtung an die gewünschte Stelle im Gastro-Intestinal-Trakt mittels eines geeigneten flexiblen Endoskops (je nach Anwendung eines Gastroskops oder eines Koloskops) werden die Hohlräume 8, 9 evakuiert, sodass sich die Ballonelemente 4, 5 unter Ausbildung von entsprechenden Faltungen an den Rohrkörper 1 anlegen. Dieser Einführzustand der Einrichtung ist in den Fig. 6 und 7 dargestellt.

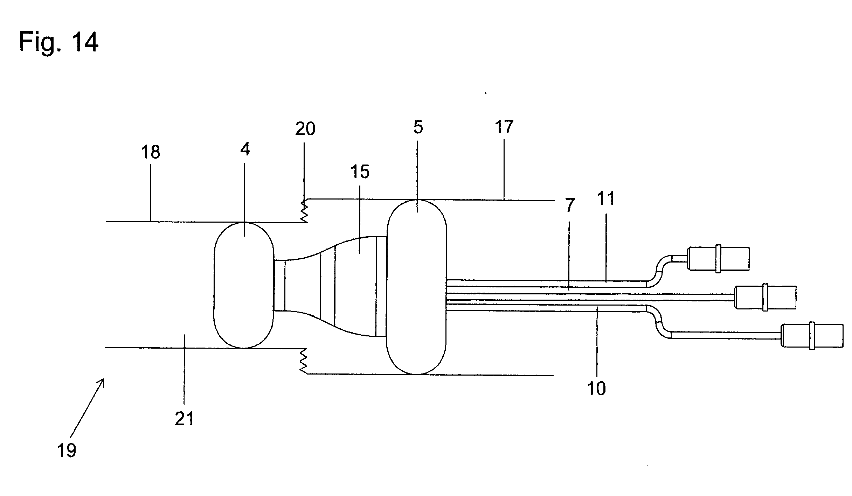

Ein mögliches Anwendungsbeispiel einer erfindungsgemäßen Einrichtung ist in Fig. 14 stark schematisiert dargestellt. Angedeutet ist eine Anastomose in Form einer Ileokolostomie, bei welcher der Dickdarm 17 nach Entfernung eines Teils desselben an den Dünndarm 18 angeschlossen wurde. Der Rohrkörper 1 erstreckt sich über den Bereich der Anastomose, wobei das erste Ballonelement 4 im Endabschnitt des Dünndarms 18 und das zweite Ballonelement 5 im Endabschnitt des Dickdarms 17 liegt. Nach der chirurgischen Platzierung der Einrichtung mittels eines Endoskops, wobei die Ballonelemente 4, 5 evakuiert sind, werden die Ballonelemente 4, 5 über die Befüllleitungen 10, 11 bis zur vorgesehenen Ausdehnung mit einem Füllmedium befüllt. Im Weiteren wird ein Unterdruck an die Drainageleitung 7 angelegt. Die im Bereich zwischen dem ersten und dem zweiten Ballonelement 4, 5 liegende Innenwand des Gastro-Intestinal-Trakts 19 wird damit, zumindest teilweise, gegen die Folie 15 gezogen. In diesem Bereich liegt die intraluminale Verletzung 20, welche hier von der chirurgischen Naht der Anastomose gebildet wird. Es wird damit ein Formschluss zwischen der Innenwand des Gastro-Intestinal-Trakts 19 und der Einrichtung ausgebildet, welcher ein Verschieben der Einrichtung in Längsrichtung des Gastro-Intestinal-Trakts 19 verhindert. Im Weiteren wird der zwischen den Ballonelementen 4, 5 liegende Bereich der Innenwand des Gastro-Intestinal-Trakts gegenüber dem Lumen 21 des Gastro-Intestinal-Trakts 19 abgedichtet. Der Bereich der Verletzung 20 wird damit gegenüber im Lumen 1 geführten Stoffen geschützt. Im Weiteren wird über das poröse Material 6 und die Drainageleitung 7 sich im Bereich der Verletzung 20 befindende oder bildende Flüssigkeit abgesaugt. Der Heilungsprozess der Verletzung 20 wird dadurch vorteilhaft unterstützt.

Die erfindungsgemäße Einrichtung ist insbesondere bei unterschiedlichen Arten von Anastomosen vorteilhaft einsetzbar. Abgesehen von einem Einsatz im Zusammenhang mit einer Ileokolostomie kann die Einrichtung beispielsweise auch im Zusammenhang mit einer Kolokolostomie oder einer Ösophago-Gastrotomie eingesetzt werden. Beispielsweise kann eine erfindungsgemäße Einrichtung auch vorteilhaft im Zusammenhang mit einer Sleevegastrektomie eingesetzt werden, um die chirurgische Klammernaht zu schützen, typischerweise im Hiss'schen Winkel.

Eine erfindungsgemäße Einrichtung kann unmittelbar nach der Operation eingesetzt werden, um das Abheilen der Verletzung durch die Operation zu unterstützen. Auch ein späterer Einsatz im Falle von Komplikationen ist vorteilhaft möglich.

Je nach vorgesehenem Einsatzort der Einrichtung ist die Einrichtung mit einer entsprechenden Größe des Rohrkörpers 1 und einem entsprechenden Außendurchmesser der Ballonelemente 4, 5 in ihrem expandierten Zustand ausgebildet. So sind die Ballonelemente 4, 5 für einen Einsatz im Magen-Darm-Trakt auf einen Außendurchmesser von mindestens 25mm expandierbar. Eine solche Expandierbarkeit auf einen Außendurchmesser von mindestens 25mm ist beispielsweise für einen Einsatz speziell im Ösophagus vorgesehen. Wenn ein Einsatz der Einrichtung speziell im Dickdarm vorgesehen ist, sind die Ballonelemente 4, 5 im gefüllten Zustand auf einen Außendurchmesser von mindestens 35mm expandierbar.

Der Rohrkörper 1 weist eine ausreichende Flexibilität auf, insbesondere um das Einführen der Einrichtung zu erleichtern. Die elastische Dehnbarkeit des Rohrkörpers 1 ist aber wesentlich geringer als diejenige der Ballonelemente 4, 5, beträgt vorzugsweise weniger als 25% der elastischen Dehnbarkeit der Ballonelemente 4, 5. Wenn der Rohrkörper 1 und die Ballonelemente 4, 5 aus dem gleichen Material bestehen, beispielsweise Silikon, so ist die Wandstärke des Rohrkörpers 1 wesentlich dicker als diejenige der Ballonelemente 4, 5, vorzugsweise mindestens doppelt so dick.

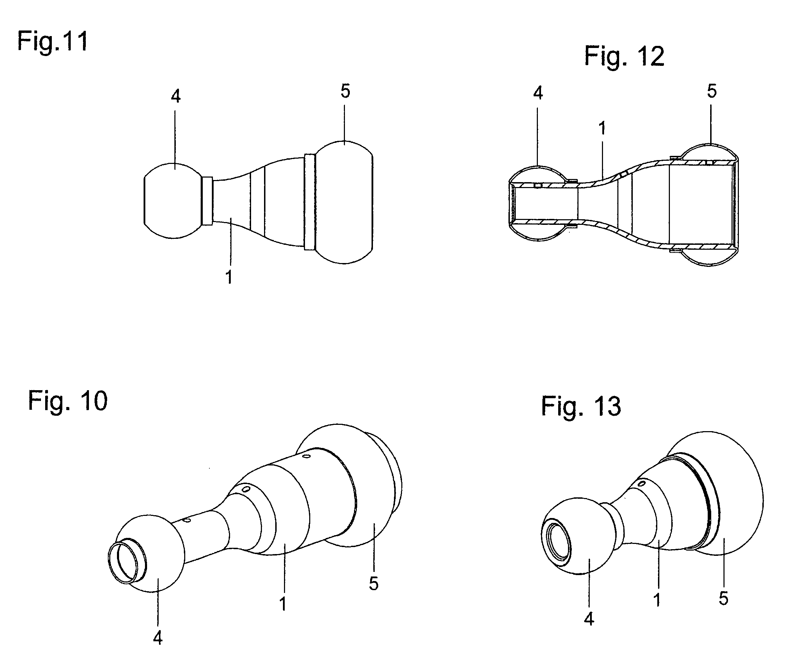

In einem vorteilhaften Herstellungsverfahren einer erfindungsgemäßen Einrichtung werden der Rohrkörper 1 und die die Ballonelemente 4, 5 bildenden Membrane in Form eines einzigen Spritzgussteils ausgebildet, vgl. Fig. 8 bis 10. Die die Ballonelemente 4, 5 bildenden Membrane sind somit an den Rohrkörper 1 angespritzt. Das Spritzgussteil 22 weist somit im Bereich der die Ballonelemente 4, 5 ausbildenden Membrane eine geringere Wandstärke als im Bereich des dem Rohrkörper 1 ausbildenden Abschnitts auf. Nach dem Spritzen stehen die die Ballonelemente 4, 5 ausbildenden Membrane zunächst von den axialen Enden des Rohrkörpers 1 axial ab, wie dies aus den Fig. 8 bis 10 ersichtlich ist. In der Folge werden sie umgestülpt, sodass sie außen über die an die axialen Enden des Rohrkörpers 1 anschließenden Abschnitte des Rohrkörpers 1 zu liegen kommen, wie dies aus den Fig. 11 bis 13 ersichtlich ist. Die zunächst freien Endabschnitte der die Ballonelemente 4, 5 bildenden Membrane werden mit der äußeren Oberfläche des Rohrkörpers 1 gas- und flüssigkeitsdicht verbunden. Im Falle der Ausbildung aus Silikon erfolgt diese Verbindung insbesondere durch eine Verklebung. Im Falle der Ausbildung aus einem verschweißbaren Material könnten stattdessen auch Schweißverbindungen hergestellt werden.

In der Folge werden die Befüllleitungen 10, 11 und die Drainageleitung 7 angebracht und das poröse Material 6 wird auf die äußere Oberfläche des Rohrkörpers 1 aufgebracht und mit dieser verbunden, beispielsweise durch Verkleben oder Verschweißen. In der Folge wird die Folie 15 über die Außenseite des porösen Materials 6 aufgebracht und mit diesem verbunden, beispielsweise durch Verkleben oder Verschweißen.

Anstelle einer Ausbildung des Rohrkörpers 1 und der Ballonelemente 4, 5 aus Silikon ist auch eine Ausbildung aus einem anderen Kunststoff denkbar und möglich, z. B. aus PE oder PU.

Auch wenn das beschriebene Herstellungsverfahren vorteilhaft ist, ist grundsätzlich auch eine Herstellung in anderer Weise denkbar und möglich. So könnten der Rohrkörper 1 und die die Ballonelemente 4, 5 bildenden Membrane zunächst separate Teile sein und im Zuge der Herstellung miteinander verbunden werden, beispielsweise durch Verklebung oder Verschweißung. Die Ballonelemente könnten dann auch als (im Längsmittelschnitt durch die Einrichtung gesehen) umfänglich geschlossene Ringballone ausgebildet sein. Im Falle der separaten Ausbildung der Ballonelemente könnte der Rohrkörper 1 und die Ballonelemente dann aus dem gleichen Material oder aus unterschiedlichen Materialien bestehen, welche die zuvor genannten Materialien umfassen können.

Der Rohrkörper 1 könnte auch mehrschichtig ausgebildet sein. Eine Schicht des Rohrkörpers könnte dann als gemeinsames Spritzgussteil mit den Ballonelementen 4, 5 ausgebildet sein oder alle Schichten des Rohrkörpers 1 könnten zunächst als von den Ballonelementen 4, 5 unabhängige Teile ausgebildet werden und eine Verbindung erst im Zuge der Herstellung erfolgen.

Unterschiedliche weitere Modifikationen der gezeigten Ausführungsform der Erfindung sind denkbar und möglich. So könnten beispielsweise mehrere Drainageleitungen 7 vorgesehen sein. Ein Abschnitt der Drainageleitung 7 könnte auch von einer Hohlkammer im Rohrkörper 1 gebildet werden. Beispielsweise könnte der Rohrkörper 1 zumindest über einen Abschnitt seiner Längserstreckung doppelwandig ausgebildet sein. Der Zwischenraum zwischen den beiden Wandungen könnte dann einen Abschnitt der Drainageleitung bilden, wobei die äußere Wand zum porösen Material 6 hin eine oder eine Mehrzahl von Öffnungen aufweisen könnte.

### Legende zu den Hinweisziffern:

- 1: Rohrkörper
- 2: Längsmittelachse
- 3: Durchgangskanal
- 4: erstes Ballonelement
- 5: zweites Ballonelement
- 6: poröses Material
- 7: Drainageleitung
- 8: Hohlraum
- 9: Hohlraum
- 10: Befüllleitung
- 11: Befüllleitung
- 12: Ventil
- 13: Ventil
- 14: Ventil
- 15: Folie
- 16: Durchtrittsöffnung
- 17: Dickdarm
- 18: Dünndarm
- 19: Gastro-Intestinal-Trakt
- 20: Verletzung
- 21: Lumen
- 22: Spritzgussteil

## Patentansprüche

1. Einrichtung zur Behandlung von intraluminalen Verletzungen des Gastro-Intestinal-Trakts, umfassend einen Rohrkörper (1) mit einem den Rohrkörper (1) in Richtung seiner Längserstreckung durchsetzenden flüssigkeitsdichten Durchgangskanal (3) und ein an der äußeren Oberfläche des Rohrkörpers (1) angeordnetes poröses Material (6), aus welchem vom porösen Material (6) aufgenommenes Fluid mittels einer Drainageleitung (7) absaugbar ist, **dadurch gekennzeichnet, dass** das poröse Material (6) nur über einem Teil der Längserstreckung des Rohrkörpers (1) an der äußeren Oberfläche des Rohrkörpers (1) angeordnet ist, dass der Rohrkörper (1) beidseitig des Teils seiner Längserstreckung, über welchen das poröse Material (6) an seiner äußeren Oberfläche angeordnet ist, jeweils von einem elastisch dehnbaren Ballonelement (4, 5) umgeben ist, dessen Außendurchmesser (a) durch Befüllen mit einem Befüllmedium vergrößerbar ist, wobei ein jeweiliges Ballonelement (4, 5) eine vorgeformte Wölbung aufweist.

2. Einrichtung nach Anspruch 1, **dadurch gekennzeichnet, dass** ein jeweiliges Ballonelement (4, 5) in Verbindung mit dem von ihm umgebenen Abschnitt des Rohrkörpers (1) einen Ringballon ausbildet, der im drucklosen Zustand einen Hohlraum (8, 9) einschließt.

3. Einrichtung nach Anspruch 1 oder 2, **dadurch gekennzeichnet, dass** ein jeweiliges Ballonelement (4, 5) im drucklosen Zustand einen größten Außendurchmesser (a) aufweist, der mindestens 50% größer ist als der Außendurchmesser des Rohrkörpers (1) im Abschnitt, über welchen das Ballonelement (4, 5) den Rohrkörper (1) umgibt.

4. Einrichtung nach Anspruch 1 bis 3, **dadurch gekennzeichnet, dass** den Ballonelementen (4,5) jeweils eine Befüllleitung (10, 11) zugeordnet ist, die von einem jeweiligen Ventil (12, 13) verschließbar ist.

5. Einrichtung nach Anspruch 4, **dadurch gekennzeichnet, dass** eine jeweilige Befüllleitung (10, 11) durch den Durchgangskanal (3) des Rohrkörpers (1) und durch eine Öffnung in der Wand des Rohrkörpers (1) verläuft.

6. Einrichtung nach einem der Ansprüche 1 bis 5, **dadurch gekennzeichnet, dass** das poröse Material (6) zur Außenseite der Einrichtung hin von einer Folie (15) abgedeckt ist, die Durchtrittsöffnungen (16) für ein Fluid aufweist.

7. Einrichtung nach einem der Ansprüche 1 bis 6, **dadurch gekennzeichnet, dass** die Ballonelemente (4, 5) jeweils direkt an das poröse Material (6) anschließen oder einen Abstand vom porösen Material (6) aufweisen, der weniger als 20% des Abstands zwischen den beidseitig des porösen Materials (6) liegenden Ballonelementen (4, 5) beträgt.

8. Einrichtung nach einem der Ansprüche 1 bis 7, **dadurch gekennzeichnet, dass** der Rohrkörper (1) im Abschnitt seiner Längserstreckung, über welchen er vom auf einer Seite des porösen Materials (6) liegenden Ballonelement (4) umgeben ist, und im Abschnitt seiner Längserstreckung, über welchen er vom auf der anderen Seite des porösen Materials (6) liegenden Ballonelement (5) umgeben ist, unterschiedliche Außendurchmesser aufweist.

9. Einrichtung nach einem der Ansprüche 1 bis 8, **dadurch gekennzeichnet, dass** die Ballonelemente (4, 5) im befüllten Zustand auf einen Außendurchmesser (a) von mindestens 25mm, vorzugsweise mindestens 35mm, expandierbar sind.

10. Einrichtung nach einem der Ansprüche 1 bis 9, **dadurch gekennzeichnet, dass** die beidseitig des porösen Materials (6) liegenden Ballonelemente (4, 5) direkt an die beiden Enden der Längserstreckung des Rohrkörpers (1) anschließen.

11. Einrichtung nach einem der Ansprüche 1 bis 10, **dadurch gekennzeichnet, dass** die Ballonelemente (4, 5) materialeinstückig mit dem Rohrkörper (1) oder zumindest einer Schicht des Rohrkörpers (1) ausgebildet sind, wobei sie eine geringere Wandstärke als der Rohrkörper (1) aufweisen.

12. Verfahren zur Herstellung einer Einrichtung nach einem der Ansprüche 1 bis 11, **dadurch gekennzeichnet, dass** ein Spritzgussteil ausgebildet wird, von welchem ein Abschnitt seiner Längserstreckung den Rohrkörper (1) oder zumindest eine Schicht des Rohrkörpers (1) bildet und von dem beidseitig an diesen Abschnitt anschließende Endabschnitte seiner Längserstreckung Membranen bilden, welche nach Umstülpen über Endabschnitte der Längserstreckung des Rohrkörpers und Verbindung ihrer zunächst freien Enden mit dem Rohrkörper die Ballonelemente (4, 5) ausbilden.

## Claims

1. Device for the treatment of internal injury to the gastrointestinal tract, comprising a tubular body (1) with a liquid-tight through-channel (3) running through the tubular body (1) in the direction of its longitudinal extension and a porous material (6) arranged on the outer surface of the tubular body (1) out of which fluid absorbed by the porous material (6) can be sucked by means of a drainage line (7), **characterised in that** the porous material (6) is only arranged on the outer surface of the tubular body (1) over a part of the longitudinal extension of the tubular body (1), that the tubular body (1) is surrounded, on each side of the part of its longitudinal extension over which the porous material (6) is arranged on its outer surface, by an elastically expandable balloon element (4, 5), the outer diameter (a) of which can be enlarged by filling it with a filling medium, wherein each balloon element (4, 5) has a pre-formed curvature.

2. Device according to claim 1, **characterised in that** each balloon element (4, 5) forms, in combination with the section of the tubular body (1) surrounding it, an annular balloon which, in an unpressurised state, encloses a cavity (8, 9).

3. Device according to claim 1 or 2, **characterised in that** each balloon element (4, 5) has, in the unpressurised state, a maximum outer diameter (a) which is at least 50% larger than the outer diameter of the tubular body (1) in the section over which the balloon element (4, 5) surrounds the tubular body (1).

4. Device according to claim 1 to 3, **characterised in that** a filling line (10, 11) is assigned to each of the balloon elements (4,5) which can in each case be closed by a valve (12, 13).

5. Device according to claim 4, **characterised in that** the respective filling lines (10, 11) run through the through-channel (3) of the tubular body (1) and through an opening in the wall of the tubular body (1).

6. Device according to one of the claims 1 to 5, **characterised in that** the porous material (6) is covered, towards the outer side of the device, by a film (15) containing openings (16) for the passage of a fluid.

7. Device according to one of the claims 1 to 6, **characterised in that** the balloon elements (4, 5) are in each case arranged directly adjacent to the porous material (6) or at a distance from the porous material (6) which amounts to less than 20% of the distance between the balloon elements (4, 5) arranged on both sides of the porous material (6).

8. Device according to one of the claims 1 to 7, **characterised in that** the outer diameter of the tubular body (1) in the section of its longitudinal extension over which it is surrounded by the balloon element (4) arranged on one side of the porous material (6) differs from that in the section of its longitudinal extension over which it is surrounded by the balloon element (5) arranged on the other side of the porous material (6).

9. Device according to one of the claims 1 to 8, **characterised in that** the balloon elements (4, 5) can be expanded in the filled state to an outer diameter (a) of at least 25mm, preferably at least 35mm.

10. Device according to one of the claims 1 to 9, **characterised in that** the balloon elements (4, 5) arranged on both sides of the porous material (6) are in direct contact with the two ends of the longitudinal extension of the tubular body (1).

11. Device according to one of the claims 1 to 10, **characterised in that** the balloon elements (4, 5) are formed of a single piece of material with the tubular body (1) or at least one layer of the tubular body (1), wherein they have a lesser wall thickness than the tubular body (1).

12. Method for manufacturing a device according to one of the claims 1 to 11, **characterised in that** an injection-moulded part is formed, a section of the longitudinal extension of which forms the tubular body (1) or at least one layer of the tubular body (1), end sections of its longitudinal extension arranged on each side of this section forming membranes which, after being folded back over end sections of the longitudinal extension of the tubular body and connection of their initially free ends with the tubular body, form the balloon elements (4, 5).

## Revendications

1. Dispositif de traitement de lésions du tractus gastro-intestinal comprenant un corps tubulaire (1), comportant un canal de passage (3) étanche aux fluides traversant ce corps tubulaire (1) dans la direction de son extension longitudinale et un matériau poreux (6) situé sur la surface externe du corps tubulaire (1) à partir duquel, un fluide recueilli par le matériau poreux (6) peut être aspiré au moyen d'une conduite de drainage (7),
**caractérisé en ce que**
le matériau poreux (6) n'est situé que sur une partie de l'extension longitudinale du corps tubulaire (1), sur la surface externe de ce corps tubulaire (1), le corps tubulaire (1) est respectivement entouré des deux côtés de la partie de son extension longitudinale sur laquelle est situé le matériau poreux (6) sur sa surface externe d'un élément en forme de ballon (4, 5) extensible élastiquement dont le diamètre externe (a) peut être augmenté par remplissage avec un fluide de remplissage, chaque élément de ballon (4, 5) comportant une courbure préformée.

2. Dispositif conforme à la revendication 1,
**caractérisé en ce que**
chaque élément formant ballon (4, 5) forme, en liaison avec le segment du corps tubulaire (1) entourant celui-ci, un ballon annulaire qui renferme, une cavité (8, 9) à l'état non pressurisé.

3. Dispositif conforme à la revendication 1 ou 2,
**caractérisé en ce que**
chaque élément formant ballon (4, 5) a, à l'état non pressurisé, un grand diamètre externe (a), qui est au moins de 50% plus supérieur au diamètre externe du corps tubulaire (1) dans le segment sur lequel cet élément formant ballon (4, 5) entoure le corps tubulaire (1).

4. Dispositif conforme à l'une des revendications 1 à 3,
**caractérisé en ce qu'**
aux éléments formant ballon (4, 5) est respectivement associée une conduite de remplissage (10, 11) pouvant être fermée par une soupape (12, 13) respective.

5. Dispositif conforme à la revendication 4,
**caractérisé en ce que**
chaque conduite de remplissage (10, 11) s'étend au travers du canal de passage (3) du corps tubulaire (1) et au travers d'une ouverture de la paroi du corps tubulaire (1).

6. Dispositif conforme à l'une des revendications 1 à 5,
**caractérisé en ce que**
le matériau poreaux (6) est recouvert, côté externe du dispositif par un film (15) qui comprend des ouvertures de passage (16) d'un fluide.

7. Dispositif conforme à l'une des revendications 1 à 6,
**caractérisé en ce que**
les éléments formant ballon (4, 5) sont chacun directement appliqués sur le matériau poreux (6) ou sont situés chacun à une distance du matériau poreaux (6) qui est égale à moins de 20% de la distance entre les éléments formant ballon (4, 5) situés de chaque côté du matériau poreux (6).

8. Dispositif conforme à l'une des revendications 1 à 7,
**caractérisé en ce que**
le corps tubulaire (1) a des diamètres externes différents dans le segment de son extension longitudinale sur lequel il est entouré par un élément formant ballon (4) situé d'un côté du matériau poreux (6) et dans le segment de son extension longitudinale sur lequel il est entouré par un élément formant ballon (5) situé de l'autre côté du matériau poreux (6).

9. Dispositif conforme à l'une des revendications 1 à 8,
**caractérisé en ce que**
les éléments formant ballon (4, 5) sont extensibles à l'état rempli à un diamètre externe (a) d'au moins 25 mm, de préférence d'au moins 35 mm.

10. Dispositif conforme à l'une des revendications 1 à 9,
**caractérisé en ce que**
les éléments formant ballon (4, 5) situés de chaque côté du matériau poreux (6) se connectent directement aux deux extrémités de l'extension longitudinale du corps tubulaire (1).

11. Dispositif conforme à l'une des revendications 1 à 10,
**caractérisé en ce que**
les éléments formant ballon (4, 5) sont réalisés en un seul matériau avec le corps tubulaire (1) ou au moins une couche du corps tubulaire (1), et ont une épaisseur de parois inférieure à celle du corps tubulaire (1).

12. Procédé de fabrication d'un dispositif conforme à l'une des revendications 1 à 11,
**caractérisé en ce que**
l'on forme une pièce moulée par injection dont au moins un segment de son extension longitudinale forme le corps tubulaire (1) ou au moins une couche du corps tubulaire (1), et dont les segments d'extrémité de son extension longitudinale se connectant de chaque côté à ce segment forment des membranes qui, après rabattement sur les segments d'extrémité de l'extension longitudinale du corps tubulaire et liaison de leurs extrémités auparavant libres avec le corps tubulaire forment les éléments formant ballon (4, 5).
